# EUROPEAN PATENT APPLICATION

(11) **EP 2 012 139 A1**
(43) Date of publication of application: **07.01.2009**
(21) Application number: 07111887.1
(22) Date of filing: 06.07.2007
(51) Int. Cl.: G01S 13/70

(54) **Body monitoring apparatus and method**

(71) Applicant: Sensino Limited, Bray, Co. Wicklow (IE)
(72) Inventor: Staderini, Enrico Maria, 1400, Yverdon-les-Bains (CH); Varotto, Graziano, 1610, Oron-la-Ville (CH)
(74) Representative: GLN

(57) **Abstract**

The present invention concerns a monitor and a method for detecting, monitoring and measuring the movement of internal body organs, such as heart, lungs, foetus, womb, etc.

The monitor comprises an Ultra Wide Band impulse transmitter, consisting of a Pseudo Random Binary Sequence generator (100), a pulse generator (101) and a transmit antenna (102), and an UWB pulse receiver, consisting of a receive antenna (106) and a boxcar averager made of a voltage controlled delay generator (113), a reference level circuit (112) and a sampler-and-averager (114). The latter is synchronously locked to the emitted pulses with a phase shift proportional to the distance between the antennas and the body organ wall to be monitored. According to the present invention, there is also provided a skin echo detector (117) for continuously determining the distance between the antennas and the skin of the patient and using it to adjust the delay of the voltage controlled delay generator to permit the latter to compensate in real time for the movements of the body.

## Description

### TECHNICAL FIELD

The present invention concerns, in general, the medical field and more particularly a monitor and a method for detecting, monitoring and measuring the movements of internal body organs, such as heart, lungs, foetus, womb, etc.

More precisely, the present invention relates to a monitor and a method, as mentioned above, making use of an impulse radar system and comprising means to compensate for the movements as a whole of the body under monitoring.

### BACKGROUND OF THE INVENTION

An impulse radar system for monitoring internal body organs is described in U.S. Patent No. 5,573,012 and its continuation in part U.S. Patent No. 5,766,208. These patents disclose (see Fig.1 of USP '012) an UWB radar consisting of an UWB (Ultra Wide Band) impulse transmitter (made of a "Pseudo Random Binary Sequence" -or PBRS- generator 100, a pulse generator 101 and a transmit antenna 102), and an UWB pulse receiver comprising a receive antenna 106 and a boxcar averager (made of a voltage controlled delay generator 113, a reference level circuit 112 and a sampler and averager 114), synchronously locked to the emitted pulses with a phase shift proportional to the time delay of the pulse-echo path to the body organ (for instance the heart) wall to be tracked. The voltage controlled delay generator receives PRBS pulses as well as a fixed delay provided by reference level circuit 112 from which it generates a sampling window (or time gating pulse) that corresponds to the measuring range of the target echo signal. No compensation is provided for the movement of the entire body so that, if the body moves, tracking of the heart wall, or of any other internal organ of interest, is extremely difficult to achieve due to the additional noise (i.e. artefacts), which is introduced into the useful signal by body movement or even impossible to achieve if, for the same reason, the echo signal arrives outside the fixed sampling window.

The prior art known in August 1994 has been extensively reviewed in the above-mentioned U.S. Patents. Other developments of - or improvements to - this technique may be found in the following documents: U.S. Patent 5,345,471, EP 694 235 and WO 04/ 057 367.

When using the apparatuses described in U.S. Patent 5,573,012 and U.S. Patent 5,766,208, the tracking of internal organ movement is greatly hampered by the movements of the body as a whole. With apparatuses of this prior art, either the system should be fixed on the body in order to maintain constant the distance between transmit and receive antennas and the body, or the body has to be kept still. In some applications, such constraints may prove to be insufferable.

So, there is still a need for a new radar system which is capable of detecting, monitoring and measuring the movement of intra-body organs and to operate, on one hand, without any contact with the body of the patient and, on the other hand, even if the body is moving with respect to said radar system.

### SUMMARY OF THE INVENTION

Accordingly, it is an object of the present invention to provide a sensing device, which overcomes the above-mentioned drawbacks of prior art systems.

It is another object of the present invention to provide a body monitoring apparatus and method, which is able to operate without any contact between the apparatus and the body.

It is still another object of the invention to provide a new impulse radar system, which is capable of detecting, monitoring and measuring intra-body movements without being limited by the movements of the body itself with respect to the radar system.

Briefly, the above and further objects and advantages of the present invention are realised by a new impulse radar system for detecting, monitoring and imaging internal body organs, which exhibits an intrinsic robustness with respect to the whole body movement. More specifically, the system of the invention includes a skin echo detector that continuously detects and permits to compensate for the variations in the distance between the skin and the system antennas. Actually, besides receiving echoes from internal body organs under monitoring, the radar system also captures the early echo from the air-skin interface and makes use of it to estimate the distance to the body in real time and to adjust the time gating pulse accordingly.

The skin echo detector adjusts the delay of the voltage controlled delay generator to correspond to the correct distance between the antennas and the body so as to maintain the tracking on the internal body organ wall of interest. The antenna-skin distance is estimated by detecting the skin echo, which is a very intense and early echo returning from the body.

### BRIEF DESCRIPTION OF THE DRAWINGS

In the following description, identical components in various figures will be designated by the same reference numbers.

The above and other features of the present invention and the manner of obtaining them will become apparent, and the invention itself will be best understood, by reference to the following description and the accompanying drawings, wherein:
FIG. 1 is a block diagram of the body monitoring and imaging apparatus of the prior art;
FIG. 2 shows a block diagram of a radar system according to the principle of the present invention;
FIG. 3 shows a block diagram of a first embodiment of the present invention;
FIG. 4 shows a timing diagram of the embodiment of Fig. 3;
FIG. 5 shows a block diagram of a second embodiment of the present invention;
FIG. 6 shows a timing diagram of the embodiment of Fig. 5;
FIG. 7 shows an implementation of the skin detector of said first embodiment; and
FIG. 8 shows an implementation of the skin detector of said second embodiment.

### DETAILED DESCRIPTION OF THE PREFERRED EMBODIMENTS

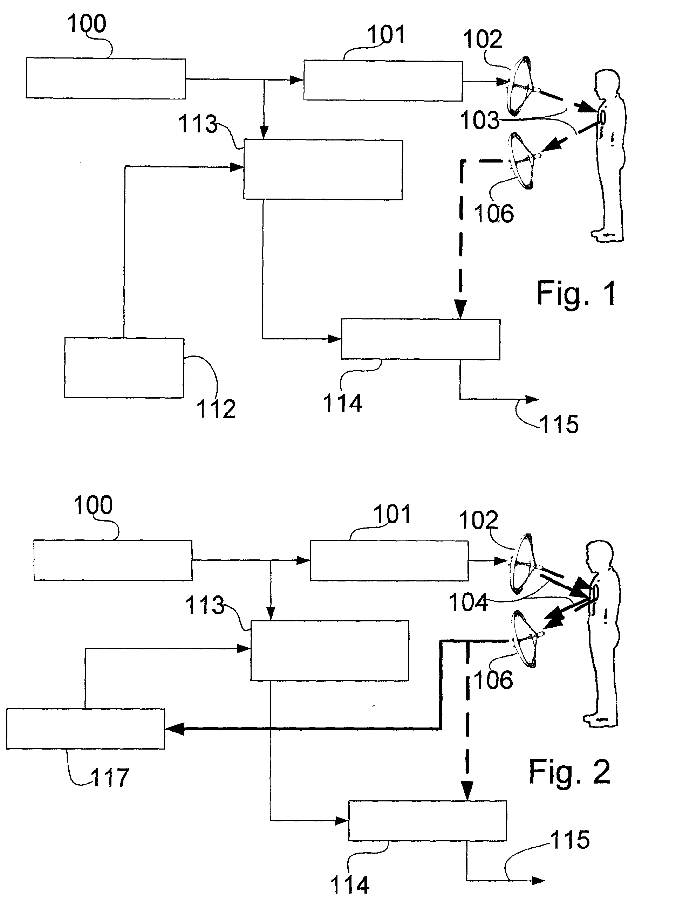

Fig. 2 shows a block diagram of a radar system in accordance with the principle of the present invention. Contrary to the prior art radar system in which the controlled delay generator 113 is controlled by a reference level circuit 112 (see Fig. 1), which gives a fixed value for establishing a fixed detection range, the system of the invention provides a skin echo detector 117. The echo signal from the receive antenna 106 is supplied to such echo detector 117 and the latter applies its output signal, which is a function of the path 104 between the skin and antennas (102 and 106), to the control input of the controlled delay generator 113 so as to adjust the range gate, i.e. the sampling window for the sampler and averager 114. This adjustment will take care of the instantaneous distance between the antennas and the body.

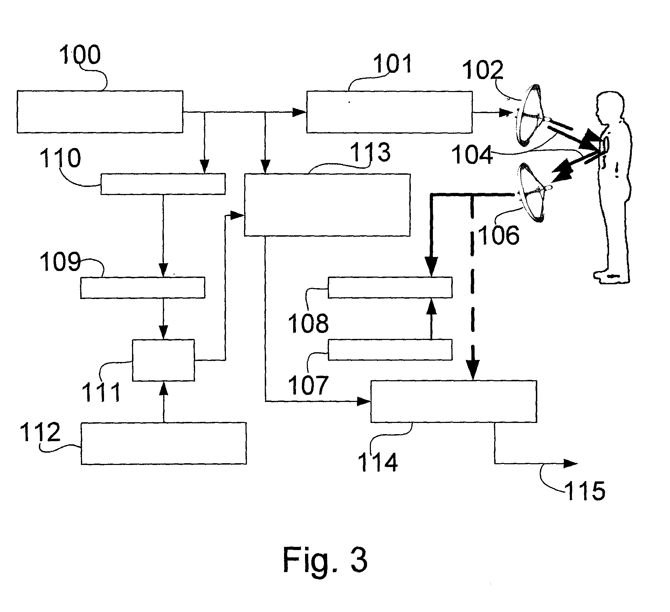

Figure 3 shows a first embodiment of the invention. The PRBS (Pseudo Random Binary Sequence) generator 100 produces a sequence of pulses with random varying interpulse period. The pulse sequence is made random - or pseudo-random - essentially for eliminating interference from other near RF (radio frequency) sources. The pulses are applied to the pulse generator 101 for producing UWB pulses (i.e. pulses of very short duration leading to a wide spectrum emitted radiation) to be radiated by the antenna 102. From the antenna the pulses can run on two paths. The shorter path 104 comprises skin reflection, while the longer path 103 considers penetration into the human body and reflection by the heart wall 105 (or any other internal organ of interest to be monitored). So the receiving antenna 106 will receive one very intense and early echo from the skin reflection and a subsequent weaker echo from the internal organ wall reflection.

A skin echo detector is composed by a comparator 108, which outputs a signal when whatsoever echo signal is higher than a predefined reference voltage level supplied by a second reference level circuit 107. The output signal of the comparator samples the voltage level provided by the ramp generator 110 by means of the sample-and-hold circuit 109. This sampled voltage, being proportional to the instantaneous distance between the skin and the antennas 102 and 106, is added by the adder 111 to a first reference voltage level provided by a first reference level circuit 112 for calibration, so that the resulting voltage is made directly proportional to the instantaneous distance of the heart wall to the antennas 102 and 106. This latter voltage is used to control the voltage controlled delay generator 113 for the operation of a boxcar averager, made of the sampler and averager circuit 114, to produce a final output signal 115. This final output signal is only proportional to the antenna-heart distance and will be further processed by processing means (not shown), such as a computer and display devices.

Compensation of body movements is thus continuously made by the skin echo detector, so as to adapt the voltage controlled delay generator to the exact distance of the internal organ wall to the two antennas. Relative distance of the body organ wall to the skin is adjusted by varying the first reference voltage level of circuit 112 while relative distance of the skin to the antennas is controlled by the second reference voltage level 107. The ramp voltage signal out of the ramp generator is continuously reset to zero at each PRBS generator pulse.

It is important to note that the first reference voltage level 112 can be negative so as to compensate even for excessive delay in response to the output signal of the comparator 108 which might happen even after the echo from the heart wall has arrived. In such a case, compensation will be effective on subsequent pulses, provided that the speed of movement of the body is very low with respect to the repetition frequency of pulses generated by the PRBS generator 100.

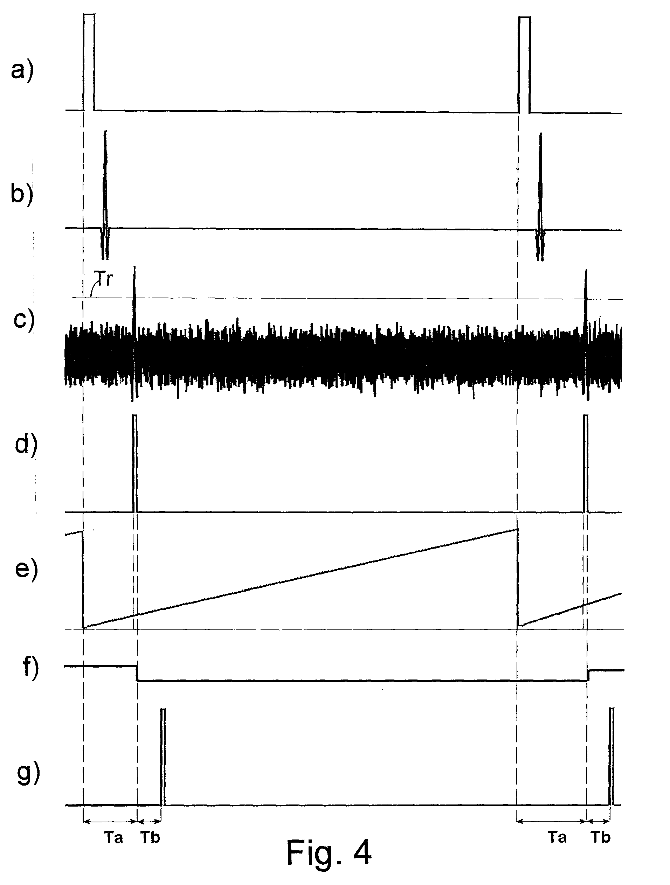

Fig. 4 shows the different waveforms, which appear in the impulse radar system of Fig. 3. Fig. 4.a shows PRBS pulses, as provided by PRBS generator 100. Typically, the average repetition frequency of these pulses is in the range of 1 to 10 MHz. Fig. 4.b shows UWB pulses generated by the pulse generator 101 from the pulses delivered by PRBS generator. As mentioned above, these pulses must have a short duration to cause the spectrum of the emitted radiation to be very large. Fig. 4.c shows the echo signal, as received by the receive antenna 106, which is applied to both the comparator circuit 108 and the sampler and averager circuit 114. As shown, the echo signal is strongly affected by noise except for peaks, which correspond to the echo signal from the skin surface and the amplitude of which is higher than the reference threshold voltage Tr that is used by the comparator circuit. The output signal of the comparator circuit, shown in Fig. 4.d, is applied to the sample-and-hold circuit 109, which also receives the ramp voltage signal, shown in Fig. 4.e, delivered by the ramp generator 110. It must be noted that the ramp generator is reset by pulses generated by the PRBS generator 100. Fig. 4.f shows the output signal of circuit 109. The latency Ta between PRBS pulses and the signal of Fig. 4.f is representative of the distance between the skin and the antennas. In order to take into account the distance between the skin and the organ surface under monitoring, a constant delay Tb, given by the first reference level circuit 112, is added to Ta by the adder circuit 111 to generate a signal, shown in Fig. 4.g, which is applied to the voltage controlled delay generator 113. As already mentioned, the latter can thus generate the sampling window for the target echo to be extracted by the sampler-and-averager circuit 114 from the echo signal (Fig. 4.c).

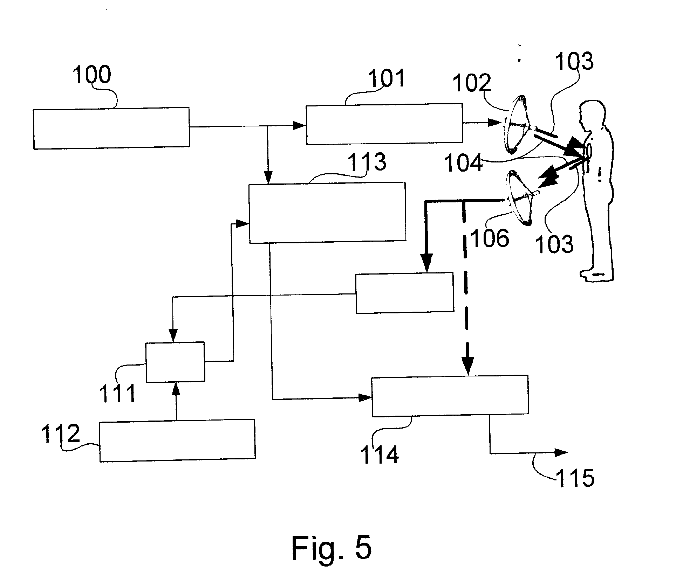

Figure 5 shows a second embodiment of the present invention. Like in the first embodiment, the impulse radar system comprises the UWB pulse transmitter, as previously described, and a UWB pulse receiver consisting of a boxcar averager 114, synchronously locked to the emitted pulse with a phase shift, proportional to the time delay to the pulse-echo path to the heart (or lung) wall to be tracked. According to the invention, a skin echo detector has been added so as to detect in a continuous way and compensate for the variation in skin-antennas distance as follows:

The skin echo detector of this second embodiment comprises a peak detector 116, the maximum amplitude of which is roughly proportional to the distance between the antenna and the skin of the patient, since the skin echo is supposed to be, not only the earliest echo signal but the one of the highest intensity. The signal out of the peak detector is adjusted to the correct compensation value, for the voltage controlled generator 113, by means of adder 111, which adds the output signal of said peak detector to the first reference voltage level provided by circuit 112. The resulting voltage is used to control the voltage controlled delay generator 113 for the operation of the boxcar averager, made of the sampler and averager circuit 114, to produce the final signal 115 out. This will only be proportional to the antennas-heart distance or M-mode heart wall signal.

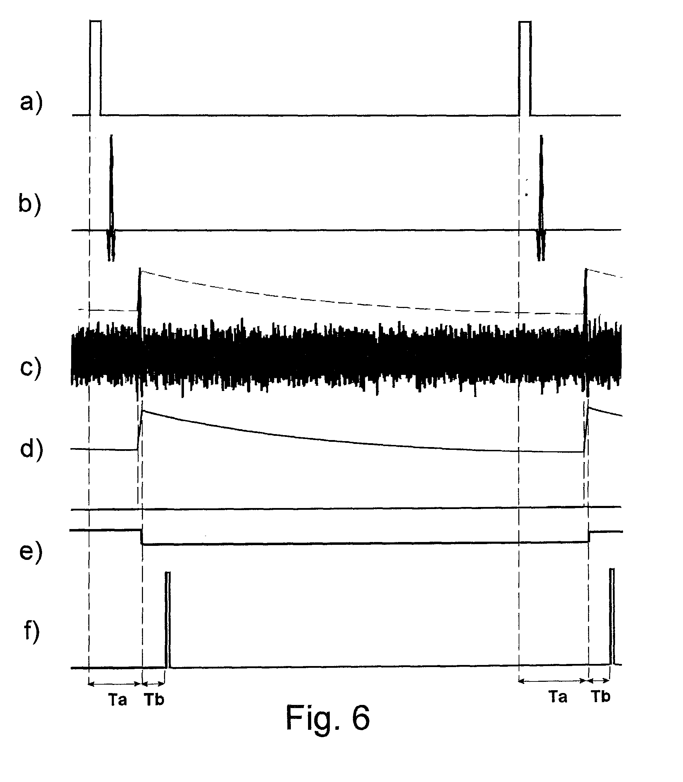

Fig. 6 shows the waveforms appearing at different points of the system of Fig. 5. Figs. 6.a, 6.b and 6.c are identical to Figs. 4.a, 4.b and 4.c and therefore, will not be described again. Fig. 6.d shows a signal generated by the peak detector 116 and Fig. 6.e shows the output signal of this peak detector. As for the first embodiment, the signal shown in Fig. 6.e exhibits a shift Ta with respect to PRBS pulses. Such shift corresponds to the path in the air between both antennas and the skin. In order to take into account the distance between the skin and the organ under monitoring, an additional constant shift Tb is added to the delay Ta through the first reference level circuit 112 and the adder circuit 111. The output signal of the adder circuit is applied to the voltage controlled delay generator, which delivers an output signal shown in Fig. 6.f to the sampler-and-averager circuit 114. As mentioned with respect to the first embodiment, the signal produced by the latter circuit may be further processed by means known to anybody skilled in the art.

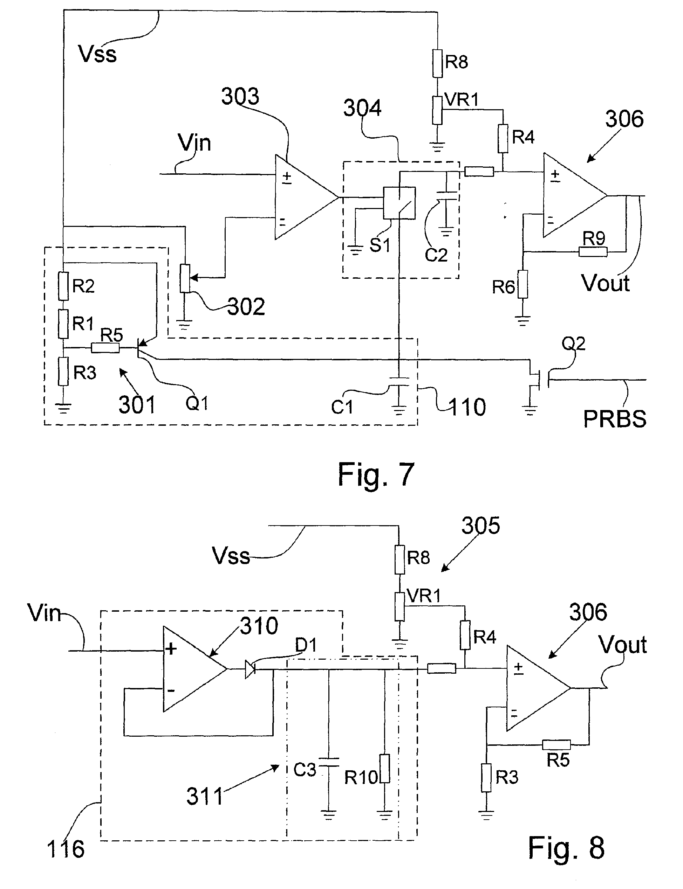

A possible implementation of the skin echo detector 117 of the first embodiment is shown in Figure 7. The signal Vin coming from the receiving antenna 106, after an amplification step which is not shown for sake of clarity, is compared by comparator 303 [ref. LT1720] with a second reference level circuit 302 (made of an adjustable resistor of 10kΩ). The output of comparator 303 drives the sample-and-hold circuit 304 (made of the switch S1 and capacitor C2 [1.8nF]), which samples the ramp signal generated by the ramp generator 110. The latter comprises a constant current source 301 (transistor Q1 [PN3640] and resistors R1 [680Ω], R2 [82Ω], R3 [3.9kΩ] and R5 [150Ω]) and a capacitor C1 [1.8nF]. The output of sample-and-hold circuit 304 is added to the first reference level provided by circuit 305 (adjustable resistor VR1 [10kΩ] and resistor R8 [1kΩ]). The resulting signal is applied to the non-inverting input of the amplifier 306 [LM6132B]. The ramp generator 110 is periodically reset by pulses from the PRBS generator through N-MOS transistor Q2 [BSH101]. When activated, the transistor Q2 rapidly discharges the capacitor C1, thus resetting said ramp generator. The output signal Vout can then drive the Voltage Controlled Delay Generator of Figure 3.

Another implementation of the skin echo detector according to the second embodiment of the invention is shown in Figure 8.

Signal Vin coming from the receiving antenna 106, after an amplification step which is not shown for sake of clarity, is applied to a zero-threshold peak detector 116. This detector consists in circuit 310 and the circuit arrangement 311; the latter comprising a capacitor C3 [1nF] and a resistor R10 [470kΩ]. Circuit 310 comprises an operational amplifier X2 [LM6132B] and a Schottky diode D1 [BAT85]. Schottky diodes are known to exhibit a very low direct voltage drop and a very fast switching time. The time constant of the circuit arrangement 311 is large enough, so that the voltage at the common node of capacitor C3 and resistor R10 is always above the noise level of the echo signal within the repetition period. As in figure 7, the output signal of the peak detector is added to the second reference level provided by circuit 305 and the resulting signal is amplified by the amplifier 306.

In the above description of both figures 7 and 8, references within brackets refer to the National Semiconductor catalogue or to the value of corresponding components.

Although the present description has been made in relation to an UWB radar system, it will be understood that it is also applicable to any other type of motion-mode radar tracing system.

Exemplary applications of the present invention may include, without any limitation;
- Foetal and uterine contraction monitoring,
- Cardiac arrhythmia monitoring,
- Pressure-pulse velocity monitoring,
- Mobile heart monitoring,
- Driver vigilance monitoring.

Changes and modifications in the specifically described embodiments can be carried out without departing from the scope of the invention, which is intended to be limited only by the scope of the appended claims.

## Claims

1. A monitor for detecting the movement of one or more body parts, comprising:
- a pulse generator (100) for producing and simultaneously inputting a sequence of pulses to a transmit path and a gating path;
- an impulse generator (101) connected to said pulse generator for producing corresponding transmit pulses;
- a transmit antenna (102) connected to said impulse generator to transmit said transmit pulses toward the one or more body parts;
- a voltage controlled delay generator (113) connected to said pulse generator for generating timed gating pulses;
- a receive antenna (106);
- a sampler-and-averager receiver (114) connected to said receive antenna in a receive path;
- said voltage controlled delay generator (113) being connected to said sampler-and-averager (114) and producing timed gating pulses at a range given by a first reference voltage circuit (112) and corresponding to the location of the one or more body parts so that said timed gating pulses cause said sampler-and-averager receiver to selectively sample pulses reflected from the one or more body parts and received by said receive antenna to produce an averaged sampled signal (115);
- processing means connected to said sampler-and-averager (114) for detecting changes in the averaged sampled signal and providing an output signal indicative of motion of said one or more body parts; and
- a skin echo detector (117) connected to the receive antenna (106), for determining the skin-antennas distance (104) and producing a driving signal (206, 214) which takes such distance into account in addition to the skin-organ distance and drives said sampler-and-averager receiver so that said gated pulses are always centered on said one or more body parts despite any variation in the distance between the body and the monitor.

2. The monitor of claim 1, wherein said transmit pulses are of the Ultra Wide Band type.

3. The monitor of any of claims 1 and 2, wherein said pulse generator has a repetition frequency in the range of 1 to 10 MHz.

4. The monitor of any of claims 1 to 3, wherein said skin detector comprises:
- a comparator (108) connected to the receive antenna and to a second reference level circuit (107) and producing a signal indicative of the distance between antennas and the skin;
- a sample-and-hold circuit (109) connected to said comparator and to a ramp generator (110) and delivering a signal representative of the antenna-skin distance;
- an adder circuit (111) connected to said sample-and-hold circuit and to a second reference level circuit (112) for adding said antenna-skin distance to the skin-organ wall distance and applying a corresponding calibration signal (206) to said voltage controlled delay generator.

5. The monitor of any of claims 1 to 3, wherein said skin detector comprises:
- a peak detector (116) connected to said receive antenna and producing a signal representative of the antenna-skin distance;
- an adder circuit (111) connected to said peak detector and to said first reference level circuit (112) for adding said antenna-skin distance to the skin-organ wall distance and applying a corresponding calibration signal (206) to said voltage controlled delay generator.

6. A method for monitoring the movement of one or more body parts, comprising:
- simultaneously inputting a sequence of pulses to a transmit path and a gating path;
- producing transmit pulses in said transmit path from said input sequence and launching said transmit pulses toward the one or more body parts;
- determining the antenna-skin distance from pulses reflected from the skin and generating time gating pulses which are phase-shifted with respect to said sequence of pulses by an amount that corresponds to said antenna-skin distance plus the distance between the skin and the one or more body parts;
- selectively receiving pulses reflected from the one or more body parts by receiving reflected signals coincident with the time gating pulses;
- sampling and storing said selected reflected pulses to produce an averaged sampled signal; and
- detecting changes in the averaged sampled signal to detect movement of the one or more body parts.
